# EUROPEAN PATENT APPLICATION

(11) **EP 1 228 686 A1**
(43) Date of publication of application: **07.08.2002**
(21) Application number: 00811245.0
(22) Date of filing: 27.12.2000
(51) Int. Cl.: A01K 11/00

(54) **Implantable identification marker**

(71) Applicant: Datamars SA, 6930 Bedano-Lugano (CH)
(72) Inventor: Stegmaier, Peter, 6946 Ponte Capriasca (CH); Bruinink, Arie, 8307 Effretikon (CH); Gwalter, Rudolf, 8645 Jona (CH); Schlosser, Viola, 8330 Pfäffikon (CH); Mayer, Jörg, 5702 Niederlenz (CH)
(74) Representative: Liebetanz, Michael, Dipl.-Phys.

(57) **Abstract**

An implantable identification marker comprises an electronic device (4) enclosed within a vial (1; 2) isolating the device (4) from body fluids of the animal after insertion of the marker into the body of an animal. The marker further comprises a fixation structure (8; 5) providing a significantly in-creased total surface of the marker and/or a reduced specific weight of the marker. This improves the integration of the marker into the surrounding tissue avoiding migration within the animal.

## Description

The invention relates, in general, to an implantable identification marker.

Such an identification marker is to be implanted subcutaneously, i.e. under the skin. It comprises an electronic device, e.g. a transponder, containing identification information about the animal which can be read by an external detector. Such implantable transponder are known and have significant utility in the biomedical field as well as for identification of domestic animals as e.g. dogs or cats.

One of the major requirements associated with manufacture of an implantable electronic transponder is the encapsulation of the IC circuit hybrid assembly and antenna coil, so that after insertion into the animal these critical components are isolated from animal body fluids. The encapsulation material must be bio-compatible and completely non-adverse to the surrounding tissue at the implant site.

It is further known, that a certain percentage of such identification markers migrate away from the initial location throughout the lifetime of the animal. It is, however, preferred to have a stationary identification marker. First of all, the reading area should be always the same within each individual animal. Furthermore, such an identification marker may migrate into areas where it hinders the animal.

US 5,074,318 discloses an anti-migration means in the form of a polymer layer coating with a high coefficient of friction. Also polypropylene is used as a coating. Furthermore the layer can be formed through partially inserting a marker in a mold cavity, injecting resin and curing it. Finally it is suggested to etch an outer glass coating of the marker.

US 5,840,148 discloses a bio-compatible anti-migration cap with two sharp projections withholding the marker in the injection device and preventing migration against the direction of insertion of the marker, i.e. the loss of the marker through the insertion wound. However, US 5,840,148, may prevent migration in one direction only and the effects of the coatings in US 5,074,318 may not be sufficient.

It is therefore an object of the invention to improve the integration of the marker into the surrounding tissue avoiding migration within the animal.

This problem is solved through a marker with the features according to claim 1.

The advantage of the marker according the invention is the possibility of interaction of the tissue surrounding the marker with the structure of the marker. This interaction is fundamentally different to the interaction with the etched glass surface, the layer coating, the cap or the pointed uni-directional projections known from the prior art.

Still other objects and advantages of the invention will be obvious or apparent from the description.

The invention is now described in view of the accompanying drawings showing different embodiments of the invention.
- Fig. 1: a schematic sectional view of a marker according to a first embodiment of the invention in its implanted state,
- Fig. 2: a view form above onto a marker constructed in accordance to a second embodiment of the invention,
- Fig. 3: a sectional view taken along line 2-2 in Fig. 2,
- Fig. 4: a sectional view of a marker according to a third embodiment of the invention before implantation,
- Fig. 5: a sectional view of a portion of a marker according to a fourth embodiment of the invention after its implantation,
- Fig. 6: a schematic sectional view of a marker according to a fifth embodiment of the invention in its implanted state, and
- Fig. 7: a schematic sectional view of an implantation set with a marker according to Fig. 1.

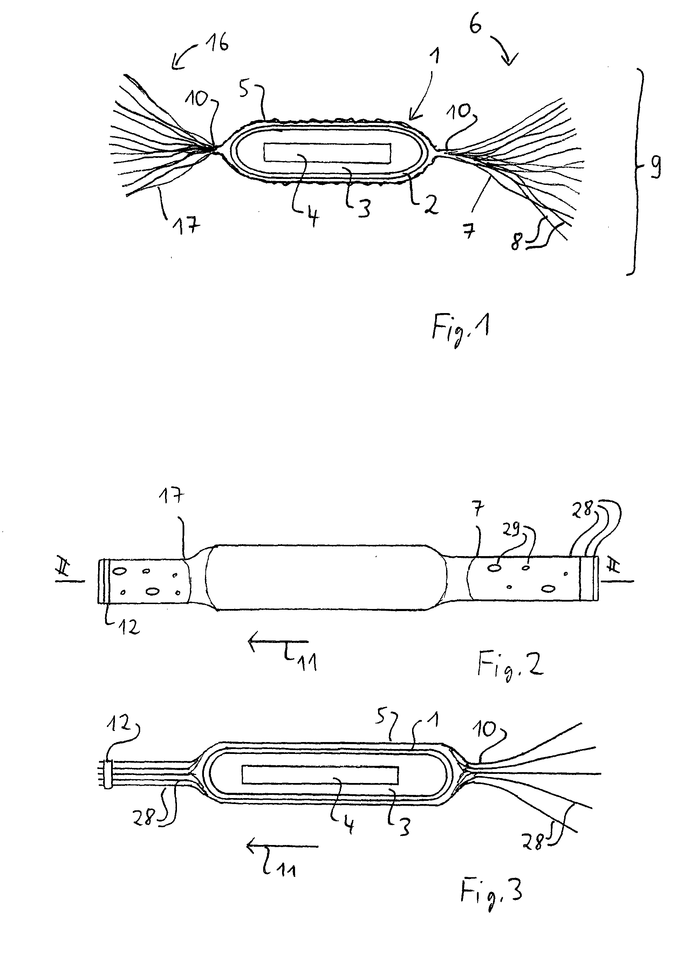

Fig. 1 is showing a schematic sectional view of a marker according to a first embodiment of the invention after insertion into an animal. Usually said markers are used for domestic animals as dogs or cats. However, it can also be used with other animals.

The marker according to Fig. 1 is composed of a bio-compatible glass vial 1 having a wall 2. The initially open glass vial 1 may be filled with a potting material 3 up to a predetermined level. Then a transponder unit 4 is introduced into the glass vial 1 and into the potting material 3. The transponder 4 can be comprised of an IC circuit and an antenna coil. It is also possible that the transponder 4 is an electronic identification device having others and/or additional functionalities.

Subsequently, the glass vial 1 is sealed. This can be performed through closing the open vial 1 with a cap, with a flame-based technology or with the use of a laser. The material 3 may also be a UV curable material in a liquid state.

Beside the use of a one-piece vial 1 as shown in Fig. 1, other embodiments may use a cap to build the gas impermeable means to protect the transponder from the environment. The transponder unit 4 is shown as box shaped.

The vial 1 is enclosed within a fine net 5, embroidery, knit or wickerwork. Although the textile net 5 may be of any material, it can be made of polypropylene filaments. Preferably the net 5 is tube-shaped with a diameter smaller then the diameter of the vial 1, so that it is biased against said vial 1.

At least at one end 6 or at both ends 6 and 16 of the vial 1 the net 5 merges into an implantation structure 7. In Fig. 1 two implantation structures 7 and 17 are provided at both ends 6 and 16 of the vial 1. Every structure 7 or 17 is preferably composed of a restricted structure 10 welded to the net 5 in order to ensure that the vial 1 stays encapsulated within the net 5. Furthermore the structures 7 or 17 comprise a multitude of single filaments 8 of a bio-compatible material, forming a cone 9. The single filaments 8 have a length of e.g. 3 to 10 millimetres with a typical length of the vial 1 of 1 to 2 centimetres. Within the cone 8, the single filaments 8, multi-filaments, textiles, ribbons, net, knit or wickerwork may have a distance one from another between 10 and 1000 micrometers. The number of filaments 8 may be between 20 and 200 and they may have a diameter between 10 and 100 micrometers. Within the body of an animal the filaments 8 of such a marker may permit blood vessels and tissue to grow into the region of the cone 9 and to encapsulate each single filament 8 to ensure the stable positioning of the implanted marker. The filaments 8 may be more or less straight (as shown in Fig. 1) or the may be curled and entwine.

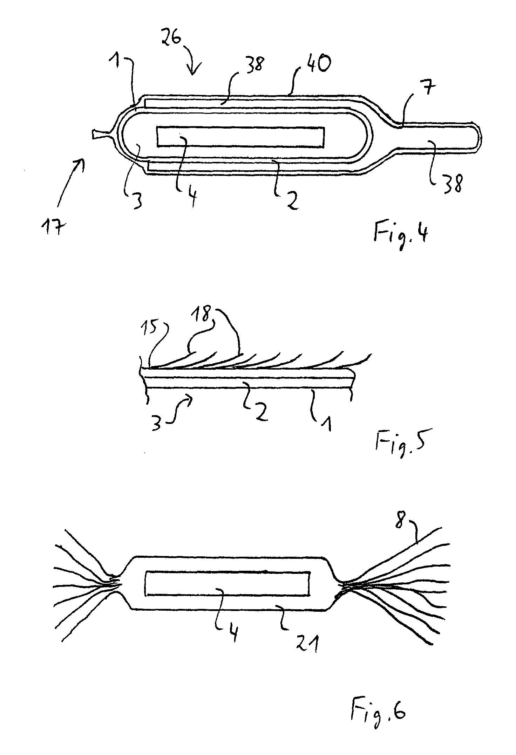

The marker to be implanted may have the single filaments 8 encapsulated within a rapidly biodegradable material, in a way that the single filaments 8 do not extend beyond the diameter of the vial 1. After the dissolution of said material, the pre-biased single filaments 8 spread and form the cone 9. It is also possible to provide a single band 12 (see Fig. 2 and 3) of biodegradable material around the ends of the single filaments 8 to ensure that the diameter of the bound single filaments 8 does not surpass the diameter of vial 1 (including the net 5). It also is possible to permit the single filaments 8 at the distal end 6 of the vial 1 to spread within the injection needle to ensure the position of the marker within said needle before insertion of the marker into the animal body. Arrow 11 shows the direction of implantation in Fig. 2 and 3.

Fig. 2 shows a schematic view from above onto a marker constructed in accordance to a second embodiment of the invention. Here the end structures 7 or 17 are composed of several sheets 28 of bio-compatible material. It can be seen from the uppermost sheet 28, shown in Fig. 2, that they contain holes 29 of different diameters. The diameter of these holes 29 can vary e.g. between 2 and 400 micrometers. Greater diameters may depend on material choice.

Fig. 3 shows a sectional view taken along line 2-2 in Fig. 2. It can be seen that there are several sheets 28, glued together in the zone 10. The number of five sheets 28 is chosen to simplify the graphical representation. Usually there are between 2 and 20 sheets 28. The sheets 28 may be pre-biased to spread after insertion of the marker into the animal. The length of the sheets 28 may be equal to the above mentioned length of the single filaments 8 of Fig. 1. The distance between two sheets 28 may vary between 10 and 1000 micrometers. Therefore the embodiment according to Fig. 3 shows the same properties as the marker according to Fig. 1. Sheets 28 may be bound together with a single band 12 of biodegradable material or glued together with such material.

Fig. 4 shows a sectional view of a marker according to a third embodiment of the invention before implantation. The marker comprises a compressible foam 38 along the vial 1 in a zone 26 and in the portions 7 beyond the vial 1. This compressible element 38, which may be provided only along the wall 2 of the vial 1 without extending any further or extending into both portions 7 and 17, can be encapsulated with a thin biodegradable membrane 40. Within the fabrication procedure of the marker the gas between vial 1 and membrane 40 can be evacuated and therefore the compressible element 38 can be compressed to e.g. one fifth to one tenth of the expanded volume. After insertion of this marker in the form shown in Fig. 4 into an animal, the membrane 40 is dissolved and the element 38 expands. This permits the surrounding tissue to grow into the structures of element 38. Furthermore the larger volume of the foam 38 reduces the specific weight of the marker towards and even below the specific weight of the surrounding tissue, so that gravitational migration effects can be avoided.

Fig. 5 shows a sectional view of a portion of a marker according to a fourth embodiment of the invention after its implantation. The wall 2 of the vial 1 is surrounded by a planiforme structure 15 possessing filaments 18 extending away from the wall 2 of the vial 1. The filaments 18 of Fig. 5 may all be orientated in one direction. They may have a length of e.g. 500 to 5000 micrometers and may be of the same material as the filaments of the first embodiment of the invention. The planiforme structure 15 surrounding the vial 1 comprises a multitude of parallel rows with similar series of filaments 18 which are orientated in opposite direction in every row. This ensures the fixation of the vial 1 in the tissue by virtue of the growth of material into the space between the different filaments 18, into the space between filaments 18 in one row and into the space between filaments 18 of different rows.

Fig. 6 shows a schematic sectional view of a marker according to a fifth embodiment of the invention in its implanted state. The embodiment uses filaments 8 as in Fig. 1. The difference between the two embodiments lies in the fact that in Fig. 6 the transponder 4 is surrounded directly by the material 21 or layered sets of material forming the filamentous tails. Said vial 1 can be omitted, if the enclosing material constitutes a reliable barrier for water vapour, i.e. the material has no water vapour permeability. This approach can be used in all shown embodiments.

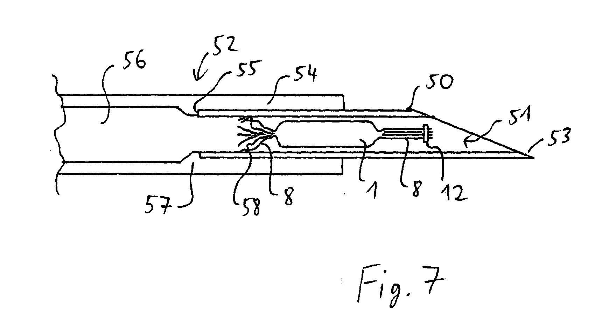

Fig. 7 shows a schematic sectional view of an implantation set with a marker according to Fig. 1. The implantation set is a needle assembly formed from a stainless steel hollow tube 50 having an exit opening 51 and an entrance opening 52. Exit opening 51 is formed in the shape of an enclined edge forming a sharp point 53 permitting the tube 50 to easily penetrate an animal's skin. Tube 50 is molded into a sleeve 54 and abuts against shoulder 55 having the same inner diameter as the hollow tube 50. In the area 56 of the sleeve beyond the hollow tube 50 the inner diameter is greater then the inner diameter of the tube 50. A tapered zone 57 forms the transitional area between the area 56 and the tube 50.

Fig. 7 shows a marker according to Fig. 3 disposed within the tube 50. The single filaments 8 of the marker directed to the exit opening 51 are bound with a single band 12 of biodegradable material to ensure that the diameter of the bound single filaments 8 does not surpass the diameter of vial 1 of the marker. The filaments 8 positioned on the other side of the vial 1 are free and can extend beyond the inner diameter of the tube 50. Therefore, they are in contact with said tube 50 at points 58 and interference fit with the inside diameter of tube 50 and prevent the displacement of the marker during storage or transport. A plunger (not shown) is slideably disposed inside the sleeve 54 and pushes the marker outside the exit opening 51 and injects it into the animal. This plunger may also be used to push the marker, preloaded in the sleeve 54, into the smaller tube 50. The tapered portion 57 between sleeve 54 and tube 50 is especially useful, when the marker is manufactured according to an embodiment according to Fig. 4 or 5.

The common point in all embodiments is the significant increase of the surface surrounding the marker upon its implantation. This is achieved through providing at least one zone 8, 18, 28, 38 with significantly increased total surface. This may also lead to an overall decrease of specific weight. This can be a cone 9 of single filaments 8, a foam 38 or expanding sheets 28. These zones can be provided at one 6 or both ends 16 of the marker, they can also be provided alongside 26 the transponder 4. In the latter case the zone is preferably initially (before implantation) compressed, since the diameter of the marker upon insertion into the animal is preferably as small as possible. The compression may be achieved through encapsulating it with a biodegradable membrane and subsequent application of a vacuum upon fabrication of the marker. This enlarges the radius of the marker by generally less than 200 micrometers. In the structures beyond the ends 6 and 16 of the transponder 4 the zones 7, 17 with significantly reduced specific weight can be pre-biased and encapsulated in a biodegradable material.

It is also possible to exert radially compressing forces upon the marker encapsulated with the material of significantly reduced specific weight to enter it into the implantation needle according to Fig. 7. Then the implantation needle with the marker is packaged. Said compressed material will ensure that the marker does not slide out of the hollow needle 50 without the action of a plunger.

The property of the zones of significantly reduced specific weight is accompanied by the surface increase within theses zones and therefore greater possibilities of interaction between the surrounding tissue and the marker. This surface increase can especially be provided through woven, knitted, braided, nonwoven and stamped fabric structures. In the case that a vial 1 of a different material as bio-compatible glass is used, it may be encapsulated by the knit, net or wickerwork tube-shaped element 5. This tube 5 is then sealed at both ends 6 an 16 of the marker by making use of a glue, pressure, heat or ultrasonic welding to create the restriction 10.

## Claims

1. An implantable identification marker comprising an electronic device (4) enclosed within a means (1, 3; 21) isolating the device (4) from body fluids of the animal after insertion of the marker into the body of an animal, comprising a fixation structure (8, 18, 28, 38) providing a significantly in-creased total surface of the marker.

2. An implantable identification marker comprising an electronic device (4) enclosed within a means (1, 3; 21) isolating the device (4) from body fluids of the animal after insertion of the marker into the body of an animal, comprising a fixation structure (8, 18, 28, 38) providing a reduced specific weight of the marker.

3. The implantable identification marker according to claim 1 or 2, wherein the electronic device is a transponder (4).

4. The implantable identification marker according to one of claims 1 to 3, wherein the fixation structure (8, 18, 28, 38) extends radially or beyond the means (1, 3; 21) isolating the electronic device (4) to permit, after insertion of the marker into the body of an animal, tissue elements of the animal to grow into said fixation structure (8, 18, 28, 38).

5. Marker according to claim 4, wherein the fixation structure (8, 18, 28, 38) extends beyond one (7) or both (17) ends of the means (1) isolating the electronic device (4).

6. Marker according to claim 5, wherein the fixation structure (8, 28) comprises a cone (9) of filaments (8) or spread sheets (28) comprising a number of holes, pores or cavities (29) of different or same diameter.

7. Marker according to one of claims 1 to 6, wherein the fixation structure (8, 28) ends in a area (10) of smaller diameter directly surrounding the means (1) isolating the electronic device (4), wherein the area (10) is part of a textile structure (5) and/or a net, enclosing the means (1) isolating the electronic device (4).

8. Marker according to one of claims 1 to 6, wherein the means (21) isolating the electronic device (4) are in one piece with the fixation structure (8, 18, 28, 38).

9. Marker according to one of claims 1 to 8, wherein the fixation structure (8, 18, 28, 38) comprises a compressible element (38) positioned on the means (1) isolating the electronic device (4) and/or beyond the means (1) isolating the electronic device (4).

10. Marker according to one of claims 1 to 9, wherein the fixation structure (8, 18, 28, 38) is enveloped by a biodegradable skin (40) or surrounded partly by said biodegradable skin (12).

11. Implantation set for a marker according to claims 1 to 10, comprising a needle portion (50) to be inserted into the skin of an animal, wherein the hollow needle portion (50) has a first inner diameter, and wherein opposite (56) to the exit opening (51) of the hollow needle portion (50) a zone (56) of a second inner diameter, larger then the first diameter, permits the loading of the marker in the needle.

12. Implantation set for a marker according to claims 1 to 10, wherein the fixation structure (8) of the marker holds the marker within the hollow needle portion (50), so that the marker can only be displaced in the needle portion through action of a plunger pushing the marker through the needle out (51) of the implantation set.
